# EUROPEAN PATENT APPLICATION

(11) **EP 3 330 708 A1**
(43) Date of publication of application: **06.06.2018**
(21) Application number: 17204361.4
(22) Date of filing: 29.11.2017
(51) Int. Cl.: G01N 33/497

(54) **BREATH ANALYZER AND METHOD FOR DETERMINING ABERRANCE IN THE SAME**

(30) Priority: 30.11.2016 JP 2016232032
(71) Applicant: Tanita Corporation, Tokyo 174-8630 (JP)
(72) Inventor: SAGAWA, Kiyoshi, Itabashi-Ku, Tokyo 174-8630 (JP); MOCHIZUKI, Kei, Itabashi-Ku, Tokyo 174-8630 (JP); FUJIE, Masahiro, Itabashi-Ku, Tokyo 174-8630 (JP)
(74) Representative: Grünecker Patent- und Rechtsanwälte PartG mbB

(57) **Abstract**

A breath analyzer (10) for determining whether aberrance in breath component measurement occurs or not, comprises: a receptacle (20) into which a breath is blown by a subject through an introduction opening (21a); a gas sensor (15) for sensing a predetermined component such as alcohol of the breath introduced into the receptacle; a pressure sensor (16) for sensing a pressure in the receptacle responding to variation of a capacity of the receptacle; a solenoid (18) for varying a capacity of the receptacle; an aberrance determination device (103) for determining whether aberrance occurs or not, based on an output of the pressure sensor responding to variation of the capacity of the receptacle by the solenoid.

## Description

### BACKGROUND OF THE INVENTION

### 1. Field of the Invention

The present invention relates to a breath analyzer and a method for determining aberrance in the same.

### 2. Description of the Related Art

JP 2014-119943A discloses a breath analyzer for measuring concentration of alcohol in breath of a subject. This breath analyzer ingests the breath of the subject into a gas sensing chamber from an introduction opening, and measures the concentration of alcohol in the breath by a gas sensor provided in the gas sensing chamber. However, if an injustice such as jamming of wadding into the introduction opening of the breath is misbehaved, an amount of the breath ingested into the gas sensing chamber through the introduction opening becomes fewer so that a measured value of the concentration of alcohol is smaller than the actual concentration of alcohol.

### SUMMARY OF THE INVENTION

The present invention is conceived in view of the above mentioned situation and purposes to provide a technology which enables to determine the injustice such as jamming of the wadding into the introduction opening of the breath.

A breath analyzer in accordance with an aspect of the present invention comprises: a receptacle having an introduction opening through which a breath of a subject is introduced and containing the breath introduced through the introduction opening; a breath component sensor for sensing a predetermined component in the breath in the receptacle; a pressure sensor for sensing a pressure in the receptacle; a capacity varying device for varying a capacity of the receptacle; and an aberrance determination device for determining whether aberrance occurs or not, based on an output of the pressure sensor responding to variation of the capacity of the receptacle by the capacity varying device.

According to this aspect, when injustice such as jamming of wadding into the introduction opening occurs, a sectional area of the introduction opening becomes smaller, so that an amount of the breath flowing into or out from the receptacle through the introduction opening becomes smaller than that in normal state that noting is jammed into the introduction opening. In other words, if injustice such as jamming of wadding into the introduction opening occurs, sealability of the receptacle becomes higher than that in the normal state that noting is jammed into the introduction opening. Higher the sealability of the receptacle becomes, larger the pressure change of the receptacle due to the variation of the capacity of the receptacle becomes. Therefore, it is possible to determine whether the injustice such as jamming of the wadding into the introduction opening of the receptacle is done (aberrance occurs) or not, based on an output of the pressure sensor responding to variation of the capacity of the receptacle by the capacity varying device.

A method for determining aberrance of a breath analyzer in accordance with an aspect of the present invention determines whether aberrance in breath component measurement occurs or not, having the steps of: introducing breath of a subject into a receptacle of the breath analyzer through an introduction opening; sensing a predetermined component in the breath in the receptacle; varying a capacity of the receptacle; and determining whether aberrance occurs or not, based on an output of a pressure sensor responding to variation of the capacity of the receptacle.

While the novel features of the present invention are set forth in the appended claims, the present invention will be better understood from the following detailed description taken in conjunction with the drawings.

### BRIEF DESCRIPTION OF THE DRAWINGS

The present invention will be described hereinafter with reference to the annexed drawings. It is to be noted that all the drawings are shown for the purpose of illustrating the technical concept of the present invention or embodiments thereof, wherein:
[FIG. 1] FIG. 1 is a perspective view showing an appearance of a breath analyzer in accordance with a first embodiment of the present invention;
[FIG. 2] FIGS. 2A to 2C are respectively a front view, a side view and a top view of the breath analyzer in accordance with the first embodiment;
[FIG. 3] FIG. 3 is a sectional front view of the breath analyzer in accordance with the first embodiment;
[FIG. 4] FIG. 4 is a block diagram showing an entire constitution of the breath analyzer in accordance with the first embodiment;
[FIG. 5] FIG. 5 is a block diagram showing a functional connection of a main controller of the breath analyzer in accordance with the first embodiment;
[FIG. 6] FIG. 6 is a graph showing a variation of output of a pressure sensor of the breath analyzer when nothing is jammed in an introduction opening of the breath analyzer in accordance with the first embodiment;
[FIG. 7] FIG. 7 is a graph showing a variation of output of a pressure sensor of the breath analyzer when wadding is jammed in an introduction opening of the breath analyzer in accordance with the first embodiment;
[FIG. 8] FIG. 8 is a flowchart showing an operation of the breath analyzer in accordance with the first embodiment;
[FIG. 9] FIG. 9 is a block diagram showing a functional connection of a main controller of a breath analyzer in accordance with a second embodiment;
[FIG. 10] FIG. 10 is a graph showing a variation of output of a pressure sensor of the breath analyzer when nothing is jammed in an introduction opening of the breath analyzer in accordance with the second embodiment;
[FIG. 11] FIG. 11 is a graph showing a variation of output of a pressure sensor of the breath analyzer when wadding is jammed in an introduction opening of the breath analyzer in accordance with the second embodiment;
[FIG. 12] FIG. 12 is a flowchart showing an operation of the breath analyzer in accordance with the second embodiment;
[FIG. 13] FIG. 13 is a block diagram showing a functional connection of a main controller of a breath analyzer in accordance with a third embodiment;
[FIG. 14] FIG. 14 is a graph showing a variation of output of a pressure sensor of the breath analyzer when nothing is jammed in an introduction opening of the breath analyzer in accordance with the third embodiment;
[FIG. 15] FIG. 15 is a graph showing a variation of output of a pressure sensor of the breath analyzer when wadding is jammed in an introduction opening of the breath analyzer in accordance with the third embodiment;
[FIG. 16] FIG. 16 is a flowchart showing an operation of the breath analyzer in accordance with the third embodiment;
[FIG. 17] FIG. 17 is a block diagram showing a functional connection of a main controller of a breath analyzer in accordance with a fourth embodiment;
[FIG. 18] FIG. 18 is a graph showing a variation of output of a pressure sensor of the breath analyzer when nothing is jammed in an introduction opening of the breath analyzer in accordance with the fourth embodiment;
[FIG. 19] FIG. 19 is a graph showing a variation of output of a pressure sensor of the breath analyzer when wadding is jammed in an introduction opening of the breath analyzer in accordance with the fourth embodiment; [FIG. 20] FIG. 20 is a flowchart showing an operation of the breath analyzer in accordance with the fourth embodiment; [FIG. 21] FIG. 21 is a graph showing a variation of output of a pressure sensor of the breath analyzer when nothing is jammed in an introduction opening of a breath analyzer in accordance with a first modified example; and
[FIG. 22] FIG. 22 is a graph showing a variation of output of a pressure sensor of the breath analyzer when wadding is jammed in an introduction opening of the breath analyzer in accordance with the first modified example.

### DETAILED DESCRIPTION OF THE EMBODIMENT

### (First Embodiment)

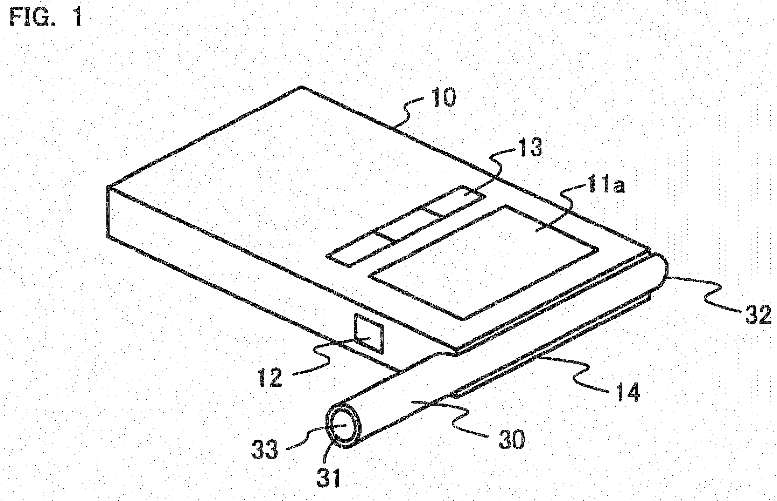

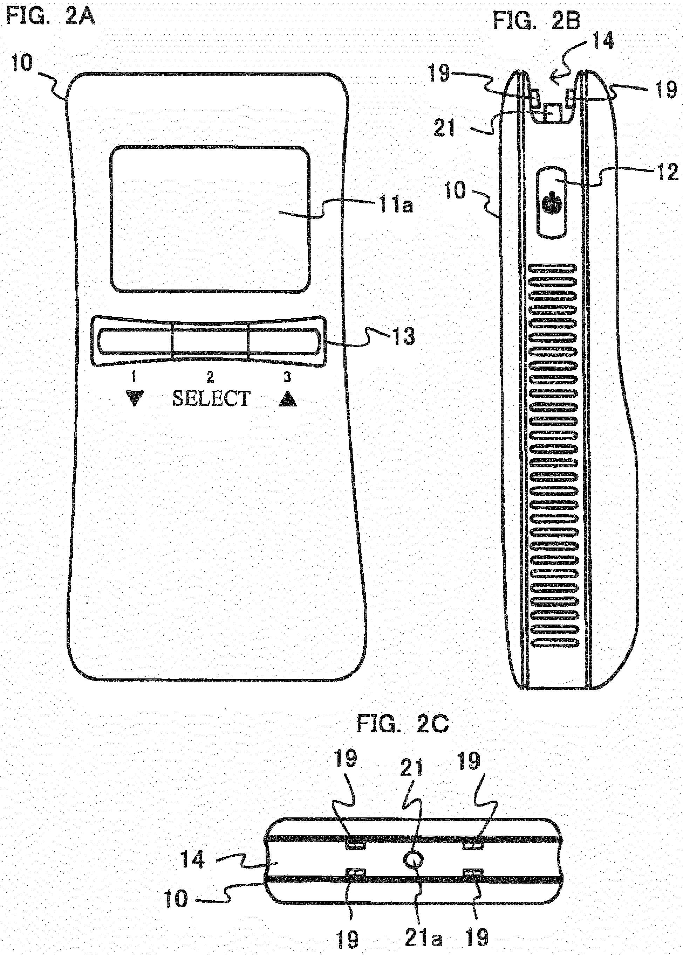

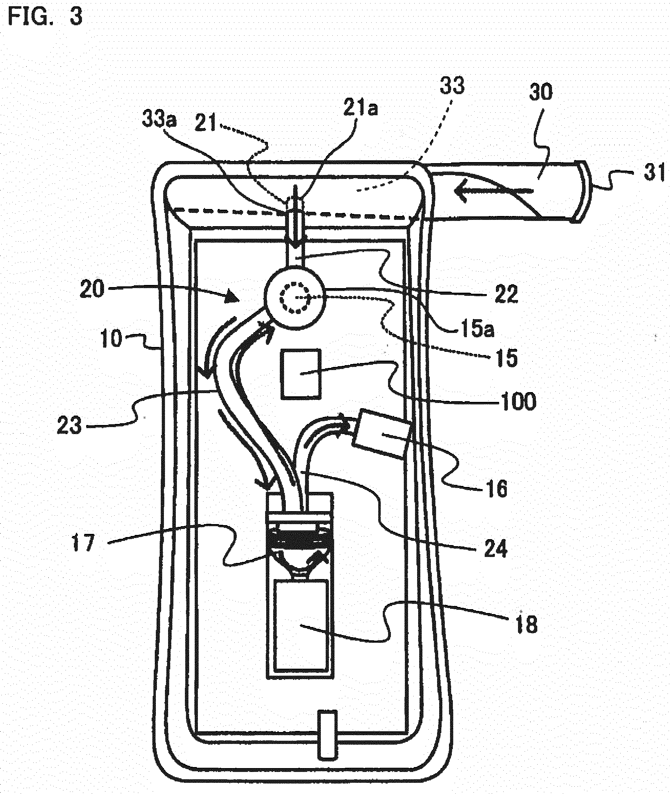

A breath analyzer and a method for determining aberrance of the breath analyzer in accordance with a first embodiment of the present invention are described with reference to the figures. FIG. 1 is a perspective view showing an appearance of a breath analyzer 10 in accordance with the first embodiment of the present invention. FIGS. 2A to 2C are respectively a front view, a side view and a top view of the breath analyzer 10, and FIG. 3 is a sectional front view of the breath analyzer 10.

As shown in FIG. 1, a mouthpiece 30 having a breath flow path 33 through which breath blown by a subject passes is detachably attached to the breath analyzer 10. The breath analyzer 10 has a power switch 12 provided on a side face, an input interface 13 configured by operation buttons or the like and provided on a front face, an attachment 14 provided on a top face by which the mouthpiece 30 is attached to the breath analyzer 10, and a display device 11a such as a LCD (Liquid Crystal Display) on the front face. As shown in FIG. 2C, a protrusion 21 is provided substantially at the center of the attachment 14 on the top face and an introduction opening 21a of a receptacle 20 is located at the center of the protrusion 21 so that the introduction opening 21 a is communicated to the breath flow path 33 of the mouthpiece 30 when the mouthpiece 30 is attached to the breath analyzer 10. The attachment 14 has four holding protrusions 19 by which the mouthpiece 30 is detachably held on the top end of the breath analyzer 10. As shown in FIG. 3, the introduction opening 21a of the receptacle 20 is substantially perpendicular to the breath flow path 33 so as to introduce the breath of the subject into the receptacle 20 provided in the inside of the breath analyzer 10. The breath analyzer 10 is a portable one including a battery such as a dry-cell battery (not shown).

The mouthpiece 30 has an inlet 31 through which the breath is blown into by the subject, an outlet 32 through which the breath is exhausted outside, and the breath flow path 33 formed between the inlet 31 and the outlet 32. A through hole 33a, with which the protrusion 21 is engaged when the mouthpiece 30 is attached to the breath analyzer 10, is formed on a side wall of the mouthpiece 30, so that the introduction opening 21a of the receptacle 20 and the breath flow path 33 of the mouthpiece 30 are communicated to each other when the protrusion 21 is engaged with the through hole 33a.

The breath analyzer 10 further comprises a gas sensor 15 for sensing a predetermined component of the breath introduced into the receptacle 20, a pressure sensor 16 for sensing a pressure in the receptacle 20, a solenoid 18 for varying a capacity of the receptacle 20, and a main processor 100 for controlling the breath analyzer 10. In this embodiment, a concentration of alcohol is measured as the predetermined component, for example.

The receptacle 20 includes a gas sensing chamber 15a into which the gas sensor 15 is provided, a bellows 17 capable of expansion and contraction, a first flow path 22 for communicating the introduction opening 21a with the gas sensing chamber 15a, a second flow path 23 for communicating the gas sensing chamber 15a with the bellows 17, and a third flow path 24 for communicating the bellows 17 with the pressure sensor 16.

The solenoid 18 serves as a part of a capacity varying device. Note that the solenoid 18, the bellows 17, and the main processor 100 correspond to claimed capacity varying device. The solenoid 18 is coupled with the bellows 17 for expanding and contracting the bellows 17. When a plunger (not shown) of the solenoid 18 moves in a first direction (for example, upward in FIG. 3), the bellows 17 is contracted so as to decrease the capacity of the bellows 17, and when the plunger of the solenoid 18 moves in a second direction (for example, downward in FIG. 3), the bellows 17 is expanded so as to increase the capacity of the bellows 17. In order to measure the predetermined component of the breath, the solenoid 18 contracts the bellows 17 first, and the solenoid 18 expands the bellows 17 subsequently, so that the breath of the subject is sucked into the receptacle 20, especially into the gas sensing chamber 15 through the introduction opening 21a from the breath flow path 33. The total capacity of the receptacle 20 is varied by expansion and contraction of the bellows 17.

The pressure sensor 16 senses the pressure in the receptacle 20. The pressure in the receptacle 20 varies responding to blowing of the breath into the breath flow path 33 by the subject and variation of the capacity of the receptacle 20. The pressure sensor 16 is configured by a semiconductor stress-strain gage adhered on a surface of a diaphragm, for example. In the pressure sensor 16, electric resistance of the gage is varied by piezoresistive effect responding to deformation of the diaphragm due to external force (pressure). The pressure sensor 16 converts variation of the electric resistances of the gages to an electric signal. Besides, the pressure sensor 16 may be another type of a pressure sensor.

The gas sensor 15 serves as a breath component sensor. The gas sensor 15 comprises a gas sensing material and measures a gas such as alcohol in the breath introduced into the gas sensing chamber 15a. In this embodiment, an electrochemical sensor having a gas sensing material is used that an electric current flows on the gas sensing material when the gas sensing material contacts to alcohol, as the gas sensor 15. A value of the electric current flowing in the gas sensing material relates to the concentration of alcohol in the breath. In the electrochemical sensor, platinum (Pt) or platinum alloy is used as a material of an anode and a cathode, and sulfuric acid (H₂SO₄) is used as an electrolyte, for example. The electrochemical sensor outputs a value responding to a variation of the electric current appearing when alcohol molecules are oxidized by the catalytic agent of platinum.

Besides, it is sufficient that the gas sensor 15 can sense alcohol in the breath. Thus, as for the gas sensor 15, various types of alcohol sensors such as a semiconductor sensor that electric resistance varies responding to concentration of alcohol in a gas or the like may be used. In the semiconductor sensor, the electric resistance of a semiconductor material varies due to reaction between oxygen adhered on a metallic oxide and alcohol in the gas.

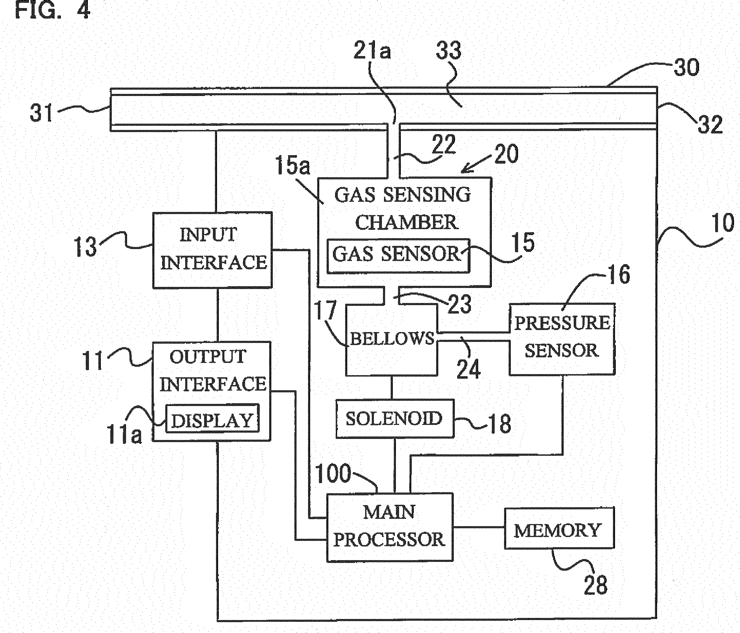

FIG. 4 is a block diagram showing the entire constitution of the breath analyzer 10. The breath analyzer 10 comprises an output interface 11 and a memory 28 further to the input interface 13, the gas sensor 15, the pressure sensor 16, the solenoid 18, the receptacle 20 (the introduction opening 21 a, the first to third flow paths 22 -24, the gas sensing chamber 15a, and the bellows 17), and the main processor 100. The output interface 11 is a device for displaying an image and/or for outputting a sound including the display device 11a, a speaker (not shown), and so on.

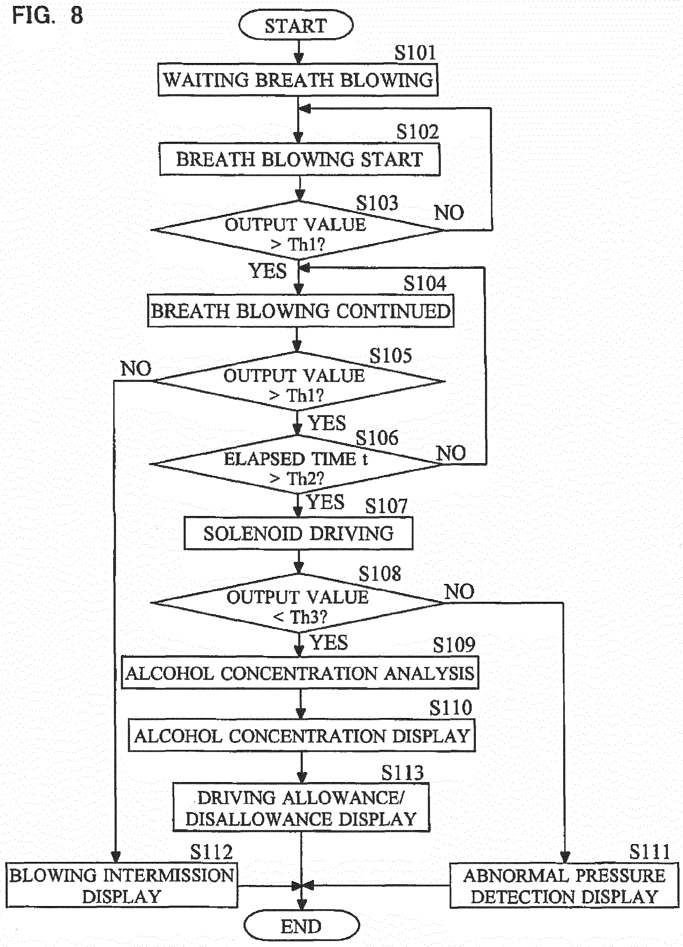

The memory 28 is a computer readable recording medium or media such as a semiconductor recording medium, a magnetic recording medium, an optical recording medium, or the like, or an optional combination of these recording media. The memory 28 stores a control program for controlling various operations of the main processor 100 such as a flow shown in FIG. 8 and various data such as threshold values Th1 to Th3, and so on.

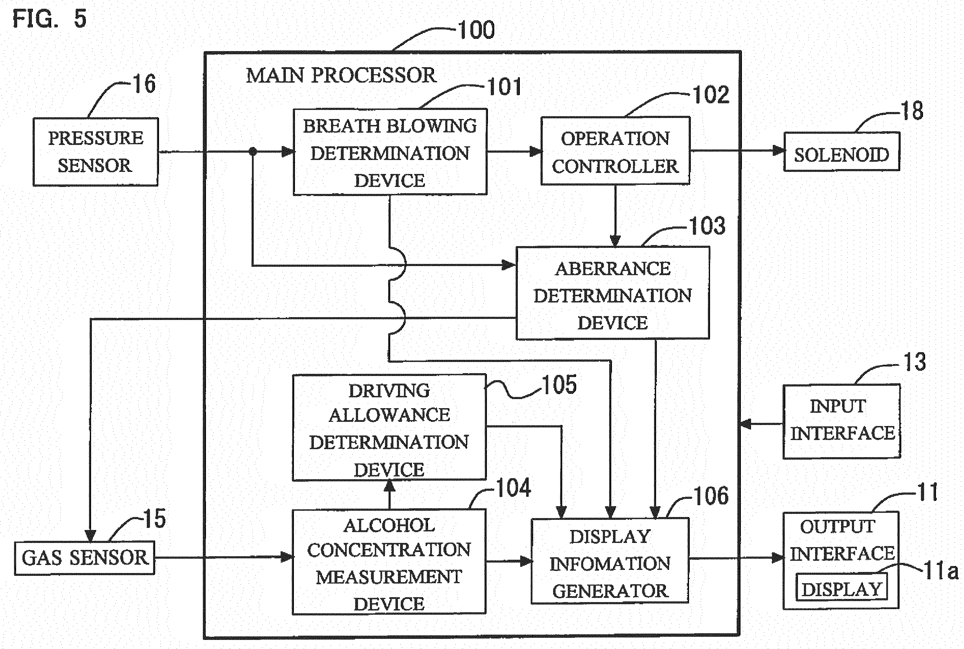

The main processor 100 is a computer constituted by a CPU (Central Processing Unit) and so on. The main processor 100 reads out the control program stored in the memory 28 and executes the control program so as to serve as a breath blowing determination device 101, an operation controller 102, an aberrance determination device 103, an alcohol concentration measurement device 104, an driving allowance determination device 105, and a display information generator 106, as shown in FIG. 5.

The breath blowing determination device 101 determines whether or not the breath is blown into the breath flow path 33, and whether or not the breath continues to be blown into the breath flow path 33 based on an output signal of the pressure sensor 16.

The operation controller 102 drives the solenoid 18 based on the result of determination by the breath blowing determination device 101. For example, when the breath blowing determination device 101 determines that a continuance time of blowing of the breath into the breath flow path 33 is longer than a threshold value Th2, the operation controller 102 outputs a solenoid control signal to the solenoid 18 for a predetermined time period and then stops outputting the signal so that the capacity of the bellows 17 is once decreased. Hereupon, the plunger of the solenoid 18 is biased to move in the second direction by a spring, for example, so that when outputting of the solenoid control signal is stopped, the plunger of the solenoid 18 will be moved in the second direction and the capacity of the bellows 17 will be increased in the meanwhile. Responding to the increase of the capacity (or expansion) of the bellows 17, the breath in the breath flow path 33 is sucked into the receptacle 20, especially into the gas sensing chamber 15a. When the operation controller 102 starts to output the solenoid control signal to the solenoid 18, it outputs driving information showing that the solenoid 18 has been driven to the aberrance determination device 103.

The aberrance determination device 103 determines whether aberrance has occurred or not based on the variation of the output of the pressure sensor 16 when the capacity of the receptacle 20 (especially the bellows 17) is varied by the operation of the solenoid 18.

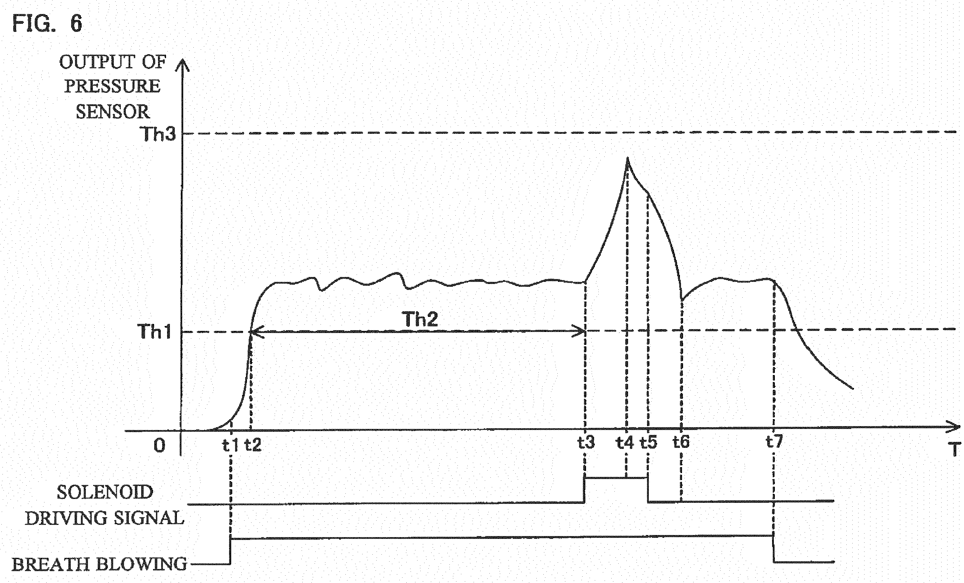

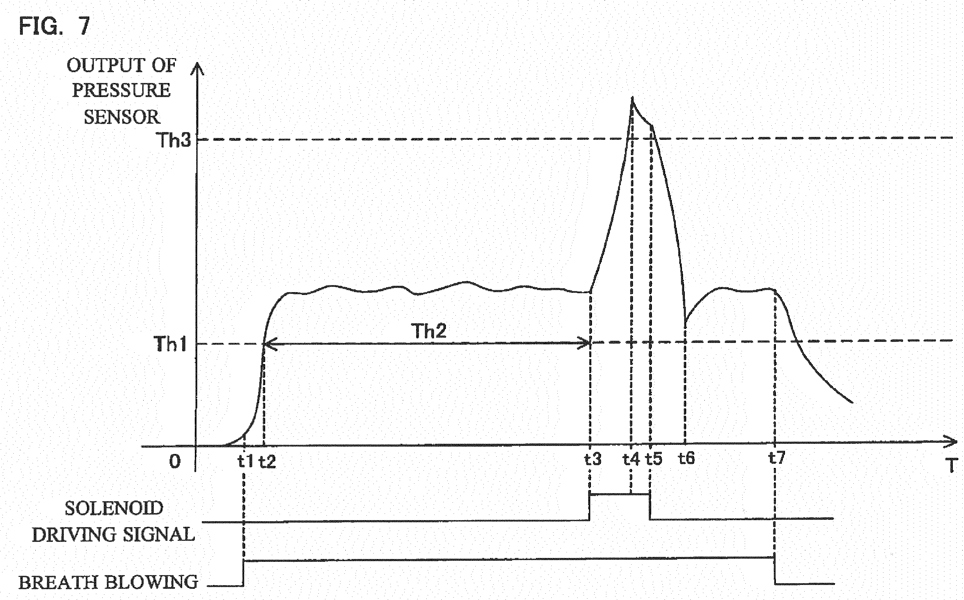

Hereupon, an aberrance state or injustice measurement due to jamming of the wadding into the introduction opening 21 a is described, as an example of the aberrance. Variation of the output value of the pressure sensor 16 due to variation of the capacity of the receptacle 20 when the wadding is jammed into the introduction opening 21 a is different from that when nothing is jammed therein. FIG. 6 shows an example of the variation of the output of the pressure sensor 16 (in other words the variation of the pressure in the receptacle 20) when the capacity of the receptacle 20 is once decreased and subsequently increased by driving the solenoid 18 under the state that noting is jammed in the introduction opening 21a of the receptacle 20. FIG. 7 shows an example of the variation of the output of the pressure sensor 16 when the capacity of the receptacle 20 is once decreased and subsequently increased by driving the solenoid 18 under the state that wadding is jammed in the introduction opening 21a of the receptacle 20.

In FIGS. 6 and 7, symbol "t1" shows the timing at which blowing of breath into the breath flow path 33 is started by a subject. Symbol "t2" shows the timing at which the output value of the pressure sensor 16 becomes larger than a threshold value Th1 due to blowing of the breath. Symbol "t3" shows the timing at which the operation controller 102 starts to output the solenoid control signal for driving the solenoid 18 when a continuance time of the output value of the pressure sensor 16 larger than the threshold value Th1 is longer than the threshold value Th2. At the timing "t3", the solenoid 18 starts to expand the plunger responding to the solenoid control signal so as to contract the capacity of the bellows 17 (or the receptacle 20) by thrust down of the bellows 17. Symbol "t4" shows the timing at which the thrust down of the bellows 17 by the solenoid 18 is completed. Symbol "t5" shows the timing at which the operation controller 102 stops outputting the solenoid control signal. The plunger of the solenoid 18 starts to move in the second direction responding to stopping the outputting of the solenoid control signal, and then the bellows 17 will be pulled in the second direction to increase the capacity thereof. Symbol "t6" shows the timing at which the pull down of the bellows is completed. Symbol "t7" shows the timing at which the blowing of the breath of the subject into the breath flow path 33 is completed.

As shown in FIG. 6 and FIG. 7, it is found that the variation of the output of the pressure sensor 16 when the wadding is jammed in the introduction opening 21a (shown in FIG. 7) is larger than that when nothing is jammed in the introduction opening 21 a (shown in FIG. 6). Since the sectional area of the introduction opening 21 a when the wadding is jammed is smaller than that when nothing is jammed, degree of sealing of the receptacle 20 or difficulty of air flowing into and out of the receptacle 20 when the wadding is jammed into the introduction opening 21a becomes higher than that when noting is jammed into the introduction opening 21a.

Then the aberrance determination device 103 determines occurrence or non-occurrence of aberrance based on the variation of the output of the pressure sensor 16. Specifically, the aberrance determination device 103 determines that aberrance occurs when the pressure detected by the pressure sensor 16 (or the output value of the pressure sensor 16) is equal to or larger than a threshold value Th3 (which is larger than the threshold value Th1) when the capacity of the bellows 17 (or the receptacle 20) is decreased by the operation of the solenoid 18, and determines that no aberrance occurs when the pressure detected by the pressure sensor 16 is smaller than the threshold value Th3. The threshold value Th3 corresponds to "first threshold value" in claim. These threshold values Th1, Th2 and Th3 are stored in the memory 28. When the aberrance determination device 103 determines that no aberrance occurs, the operation controller 102 activates the gas sensor 15 to sense alcohol in the breath sucked into the gas sensing chamber 15a. The alcohol concentration measurement device 104 measures the concentration of alcohol in the breath based on the output current value of the gas sensor 15.

The driving allowance determination device 105 determines whether the subject is allowed to drive a vehicle or not based on the concentration of alcohol in the breath calculated by the alcohol concentration measurement device 104. Specifically, the driving allowance determination device 105 determines that the subject is allowable to drive the vehicle when the concentration of alcohol in the breath is equal to or lower than a driving allowance determination threshold value stored in the memory 28.

The display information generator 106 generates data for displaying various kinds of information on the display device 11a. The display information generator 106 displays operation guide information such as instructions to wait blowing of the breath, to start the blowing of the breath, to stop the blowing of the breath, and so on, on the display device 11a. In addition, the display information generator 106 displays the value of the concentration of alcohol in the breath, result of determination of the driving allowance determination device 105, result of determination of the breath blowing determination device 101, result of determination of the aberrance determination device 103, and so on, on the display device 11 a.

Subsequently, the operation of the breath analyzer 10 in accordance with the first embodiment is described with reference to the flowchart shown in FIG. 8. First, when the subject presses down the power switch 12, the display information generator 106 displays an instruction to urge the subject to wait the blowing of the breath on the display device 11 a in a predetermined waiting time period (for example, five seconds) which is necessary to enable the measurement of the concentration of alcohol in the breath (step S101).

After passing the predetermined waiting time period, the display information generator 106 displays an instruction to start the blowing of the breath into the inlet 31 of the mouthpiece 30 during a predetermined time period (for example, five seconds) on the display device 11a (step S102). When the subject starts to blow the breath into the inlet 31 of the mouthpiece 30 responding to the instruction for encouraging the blowing of the breath, the breath passes through the breath flow path 33. At this time, a pressure of the breath is transmitted to the receptacle 20 through the introduction opening 21 a, and sensed by the pressure sensor 16.

When a variation of the pressure in the receptacle 20 is sensed by the pressure sensor 16, the breath blowing determination device 101 calculates the pressure in the receptacle 20 based on the output of the pressure sensor 16. When the output value of the pressure sensor 16 (or the pressure in the receptacle 20) is equal to or smaller than the threshold value Th1 (No in step S103), the operation of the breath analyzer 10 is returned to the step S102.

In contrast, when the output value of the pressure sensor 16 (or the pressure in the receptacle 20) is larger than the threshold value Th1 (Yes in step S103), the breath blowing determination device 101 outputs information showing detection of the blowing of the breath to the display information generator 106. Then, the display information generator 106 displays a message showing continuation of the blowing of breath on the display device 11a (step S104).

When the output value of the pressure sensor 16 (or the pressure in the receptacle 20) is equal to or smaller than the threshold value Th1 after outputting the information showing detection of the blowing of the breath (No in step S105), the breath blowing determination device 101 outputs information showing intermission of the blowing of the breath to the display information generator 106. When receiving the information showing intermission of the blowing of the breath, the display information generator 106 displays error information due to the intermission of the blowing of breath on the display device 11a (step S112).

When the output value of the pressure sensor 16 (or the pressure in the receptacle 20) is larger than the threshold value Th1 after outputting the information showing detection of the blowing of the breath (Yes in step S105), the breath blowing determination device 101 further determines whether an elapsed time period "t" is longer than a predetermined threshold value Th2 or not (step S106). Here, the elapsed time period "t" shows a time period during which the output value of the pressure sensor 16 (or the pressure in the receptacle 20) is continuously maintained larger than the threshold value Th1 from the timing at which the output value of the pressure sensor 16 (or the pressure in the receptacle 20) has been determined larger than the threshold value Th1 in the step S103. If the elapsed time period "t" is equal to or shorter than the threshold value Th2 (No in step S106), the operation of the breath analyzer 10 is returned to the step S104.

When the elapsed time period "t" is longer than the threshold value Th2 (Yes in step S106), the breath blowing determination device 101 outputs start information showing starting the sucking of the breath to the operation controller 102. When receiving the start information, the operation controller 102 outputs the solenoid control signal to the solenoid 18 for a constant time period in order to drive the solenoid 18 so that the capacity of the receptacle 20 (or the bellows 17) is once decreased and then increased (step S107). When the operation controller 102 starts the driving of the solenoid 18, it outputs driving information to the aberrance determination device 103.

Responding to the decrease of the capacity of the receptacle 20 (or the bellows 17), at least a part of the gas in the receptacle 20 is exhausted from the introduction opening 21 a. Subsequently, the breath in the breath flow path 33 is sucked into the receptacle 20 (especially, into the gas sensing chamber 15) through the introduction opening 21a responding to the increase of the capacity of the bellows 17. Here, if wadding is jammed into the introduction opening 21 a, the sectional area of the introduction opening 21a becomes smaller than that when nothing is jammed into the introduction opening 21 a, so that a flow rate of gas (breath and so on) per a unit time going into or out from the receptacle 20 becomes fewer. Due to the difference of the flow rate of the gas, the variation of the pressure in the receptacle 20 responding to the variation of the capacity of the receptacle 20 is different depending on whether or not the wadding exists in the introduction opening 21a.

The aberrance determination device 103 determines whether the output value of the pressure sensor 16 is smaller than a threshold value Th3 or not, when it receives the driving information (step S108). Specifically, when a first specified time period (for example a time period from the timing "t3" to the timing "t4" in FIGS. 6 and 7) has passed from the timing at which the driving information is outputted, the aberrance determination device 103 determines whether the output value of the pressure sensor 16 is maintained in a state smaller than the threshold value Th3 or not. The first specified time period may be set based on a calculated time period when the breath analyzer 10 is designed or an actually measured time when the breath analyzer 10 is manufactured.

When the wadding is jammed into the introduction opening 21 a, the sectional area of the introduction opening 21a becomes narrower, so that the output value of the pressure sensor 16 becomes equal to or larger than the threshold value Th3 responding to the decrease of the capacity of the receptacle 20 (or the bellows 17) as shown in FIG. 7. In contrast, when nothing is jammed into the introduction opening 21 a, the output value of the pressure sensor 16 is maintained in the state lower than the threshold value Th3 even when the capacity of the receptacle 20 (or the bellows 17) is decreased as shown in FIG. 6.

When the output value of the pressure sensor 16 is equal to or larger than the threshold value Th3 (No in step S108), that is, when the wadding is jammed into the introduction opening 21 a, for example, the aberrance determination device 103 determines that the aberrance occurs, and outputs information showing detection of abnormal pressure to the display information generator 106. When receiving the information showing the detection of abnormal pressure, the display information generator 106 displays a message showing the detection of abnormal pressure on the display device 11 a (step S111).

In contrast, when the output value of the pressure sensor 16 is smaller than the threshold value Th3 (Yes in step S108), that is, when nothing is jammed into the introduction opening 21 a, the aberrance determination device 103 determines that aberrance does not occur. Then, the operation controller 102 activates the gas sensor 15. The gas sensor 15 senses alcohol in the breath sucked into the gas sensing chamber 15a and outputs the result of sensing to the alcohol concentration measurement device 104. The alcohol concentration measurement device 104 analyzes the concentration of alcohol in the breath based on the output of the gas sensor 15 (step S109). At this time, the alcohol concentration measurement device 104 may output information showing the starting of analysis, and when receiving the information showing the starting of analysis, the display information generator 106 may display instruction to stop blowing the breath on the display device 11a. When the analysis of the concentration of alcohol in the breath is completed, the alcohol concentration measurement device 104 outputs the analysis result of the concentration of alcohol to the display information generator 106 and the driving allowance determination device 105. When receiving the analysis result of the concentration of alcohol, the display information generator 106 displays the concentration of alcohol corresponding to the analysis result on the display device 11a (step S110).

When the concentration of alcohol is equal to or larger than a threshold value of driving allowance determination, the driving allowance determination device 105 determines that the driving by the subject is not allowable, and outputs information showing that driving is not allowable to the display information generator 106. In contrast, when the concentration of alcohol is smaller than the threshold value of driving allowance determination, the driving allowance determination device 105 determines that the driving by the subject is allowable, and outputs information showing that driving is allowable to the display information generator 106. The threshold value of driving allowance determination is stored in the memory 28. When receiving the information showing that driving is not allowable, the display information generator 106 displays a message showing that driving is not allowable on the display device 11 a. On the other hand, when receiving the information showing that driving is allowable, the display information generator 106 displays a message showing that driving is allowable on the display device 11 a (step S113). Here, the message showing that driving is not allowable is an example of information corresponding to result of the aberrance determination by the aberrance determination device 103.

According to this first embodiment, occurrence or non-occurrence of aberrance in breath analysis operation is determined based on the output value of the pressure sensor 16 (or pressure in the receptacle 20) responding to the variation of the capacity of the receptacle 20. Thus, injustice such as jamming of wadding in the introduction opening 21 a of the breath analyzer 20 can be determined. In addition, the sucking of the breath into the receptacle 20 through the introduction opening 21a in order to measure the concentration of alcohol is concurrently operated to vary the capacity of the receptacle 20 in order to determine occurrence or non-occurrence of aberrance. Consequently, the driving of the solenoid 18 becomes fewer and electric power saving is enabled, in comparison with that in the case that the sucking of the breath and the variation of the capacity of the receptacle are independently operated. Furthermore, the output value of the pressure sensor 16, which is essentially used to determine the start of sucking of the breath, is used to determine occurrence or non-occurrence of aberrance. Consequently, the constitution of the breath analyzer 10 can be simplified in comparison with that in the case having a pressure sensor to determine the start of sucking of the breath and a pressure sensor to determine occurrence or non-occurrence of aberrance, independently.

### (Second Embodiment)

A breath analyzer 10 in accordance with a second embodiment of the present invention is described. In the above mentioned first embodiment, it is determined that the aberrance occurs when the output value of the pressure sensor 16 (or the pressure in the receptacle 20) becomes larger than the threshold value Th3 responding to the decrease of the capacity of the receptacle 20. In the second embodiment, occurrence or non-occurrence of the aberrance is determined based on a time period from a timing at which the output value of the pressure sensor 16 becomes larger than a threshold value Th4 to a timing at which the output value of the pressure sensor 16 becomes equal to or smaller than a threshold value Th4, when the operation to decrease the capacity of the receptacle 20 is performed. Here, the threshold values Th1, Th3 and Th4 satisfy the relation Th1 < Th4 < Th3.

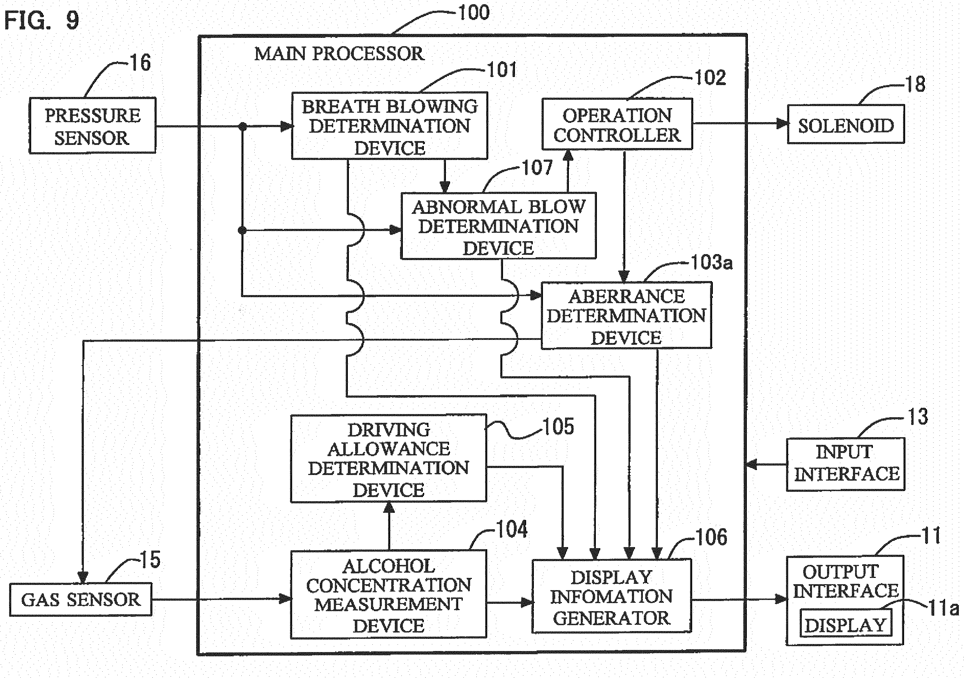

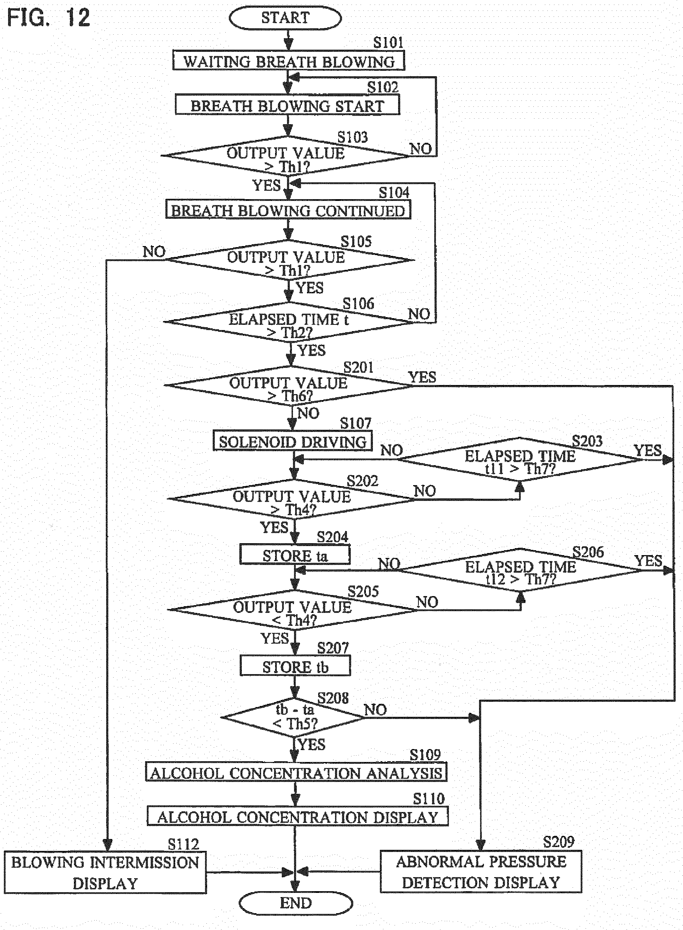

A constitution of the breath analyzer 10 in accordance with the second embodiment is different from that in the first embodiment at the following points. First, the memory 28 stores a control program for controlling various operations of the main processor 100 such as a flow shown in FIG. 12 and various data such as threshold values Th4 to Th7, instead of the control program such as the flow shown in FIG. 8 and the threshold value Th3. Second, the main processor 100 further serves as an abnormal blow determination device 107 as shown in FIG. 9. In addition, determination process in the aberrance determination device 103a in FIG. 9 is different from that in the above mentioned aberrance determination device 103 in FIG. 5 as described below.

FIG. 9 shows a functional block of the main processor 100 in the second embodiment. In the second embodiment, the main processor 100 reads out the control program stored in the memory 28 and executes the control program so as to serve as a breath blowing determination device 101, an operation controller 102, an aberrance determination device 103a, an alcohol concentration measurement device 104, an driving allowance determination device 105, a display information generator 106, and an abnormal blow determination device 107.

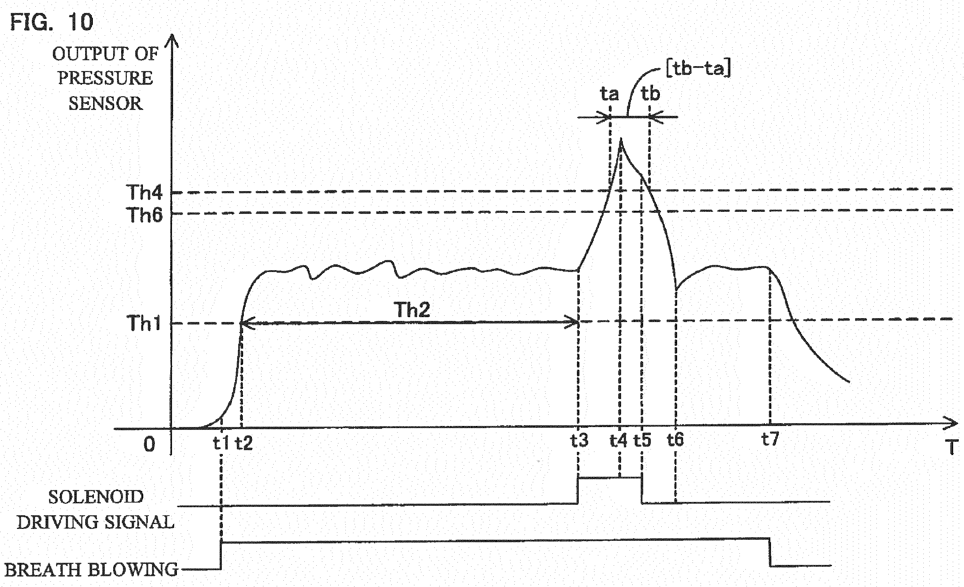

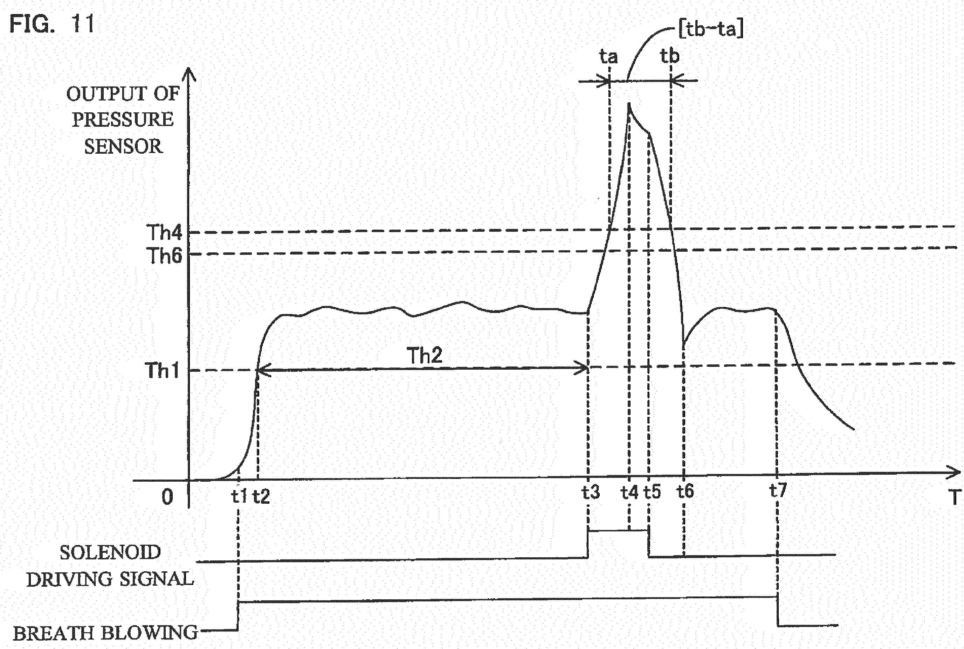

FIG. 10 shows an example of the variation of the output of the pressure sensor 16 (in other words the variation of the pressure in the receptacle 20) when the capacity of the receptacle 20 is once decreased and subsequently increased by driving the solenoid 18 under the state that noting is jammed in the introduction opening 21 a of the receptacle 20. FIG. 11 shows an example of the variation of the output of the pressure sensor 16 when the capacity of the receptacle 20 is once decreased and subsequently increased by driving the solenoid 18 under the state that wadding is jammed in the introduction opening 21a of the receptacle 20.

In FIGS. 10 and 11, symbol "ta" shows the timing at which the output value of the pressure sensor 16 becomes larger than a threshold value Th4 during the decrease of the capacity of the receptacle 20. Symbol "tb" shows the timing when the output value of the pressure sensor 16 becomes smaller than a threshold value Th4 after completion of the decrease of the capacity of the receptacle 20.

The fewer the flaw rate of gas (breath) per a unit time which goes into and out from the receptacle 20 (hereinafter abbreviated as "flaw rate of gas") becomes, the longer the time period defined by "tb-ta" (hereinafter it is called "first variation time period") becomes. The flaw rate of gas becomes fewer in proportion to the narrowness of the sectional area of the introduction opening 21 a of the receptacle 20. Therefore, the narrower the sectional area of the introduction opening 21a becomes, the longer the first variation time period becomes. If wadding is jammed into the introduction opening 21a and thus the sectional area of the introduction opening 21 a becomes narrower, the first variation time period becomes longer than that when nothing is jammed in the introduction opening 21a.

The aberrance determination device 103a determines that aberrance occurs when the first variation time period is equal to or longer than a threshold value Th5, and determines that no aberrance occurs when the first variation time period is shorter than a threshold value Th5. The threshold value Th4 corresponds to the second threshold value and the third threshold value in claim and the threshold value Th5 corresponds to the fourth threshold value in claim.

When the blowing of the breath into the breath flow path 33 is too strong and a pressure much larger than the pressure corresponding to the threshold value Th4 acts on the pressure sensor 16 only by the blowing of the breath before driving the solenoid 18, the aberrance determination device 103a cannot determine occurrence or non-occurrence of aberrance based on the output value of the pressure sensor 16. Then, the abnormal blow determination device 107 determines that an abnormal blow occurs, in the case where the output value of the pressure sensor 16 is larger than a threshold value Th6 when the subject blows the breath into the breath flow path 33. The threshold value Th6 satisfies the relation Th1 < Th6 < Th4. The threshold value Th6 corresponds to claimed ninth threshold value.

Subsequently, the operation of the breath analyzer 10 in accordance with the second embodiment is described with reference to the flowchart shown in FIG. 12. In FIG. 12, the same processes as those in FIG. 8 are designated by the same symbols. Differences from FIG. 8 are described below.

When the elapsed time period "t" becomes longer than the threshold value Th2 (Yes in step S106), the breath blowing determination device 101 outputs information showing starting to determine abnormal blow to the abnormal blow determination device 107. When receiving the information showing starting to determine abnormal blow, the abnormal blow determination device 107 determines whether the output value of the pressure sensor 16 (or the pressure in the receptacle 20) is larger than the threshold value Th6 or not (step S201).

When the output value of the pressure sensor 16 is larger than the threshold value Th6 (Yes in step S201), the abnormal blow determination device 107 determines that an abnormal blow occurs, and outputs information showing detection of abnormal blow to the display information generator 106. Receiving the information showing detection of abnormal blow, the display information generator 106 displays a message showing the occurrence of abnormal state (specifically the abnormal blow) on the display device 11 a (step S209). In contrast, when the output value of the pressure sensor 16 is equal to or smaller than the threshold value Th6 (No in step S201), the abnormal blow determination device 107 determines that a normal blow occurs, and outputs information showing starting the suction of the breath into the receptacle 20 to the operation control device 102. Receiving the information showing starting the suction of the breath, the operation control device 102 drives the solenoid 18 so that the capacity of the receptacle 20 (or the bellows 17) once decreases and then increases (step S107). When driving of the solenoid 18 starts, the operation control device 102 outputs the driving information to the aberrance determination device 103a.

When receiving the driving information, the aberrance determination device 103a determines whether the output value of the pressure sensor 16 is larger than the threshold value Th4 or not (step S202). When the output value of the pressure sensor 16 is equal to or smaller than the threshold value Th4 (No in step S202), the aberrance determination device 103a determines whether an elapsed time period "t11" from a timing at which the driving information is outputted is longer than a threshold value Th7 or not (step S203). When the elapsed time period "t11" is equal to or shorter than a threshold value Th7 (No in step S203), the operation of the breath analyzer 10 is returned to step S202. In contrast, when the elapsed time period "t11" is longer than a threshold value Th7 (Yes in step S203), the aberrance determination device 103a determines that an abnormal pressure occurs due to abnormal driving of the solenoid 18 or the like, and outputs information showing detection of the abnormal pressure to the display information generator 106. When receiving the information showing detection of the abnormal pressure, the display information generator 106 displays a message showing detection of the abnormal pressure on the display device 11a (step S209).

When the output value of the pressure sensor 16 is larger than the threshold value Th4 (Yes in step S202), the aberrance determination device 103a stores the timing "ta" at which the output value of the pressure sensor 16 becomes larger than the threshold value Th4 into the memory 28 (step S204).

Subsequently, the aberrance determination device 103a determines whether the output value of the pressure sensor 16 is smaller than the threshold value Th4 or not (step S205). When the output value of the pressure sensor 16 is equal to or larger than the threshold value Th4 (No in step S205), the aberrance determination device 103a determines whether an elapsed time period "t12" from a timing at which the timing "ta" is stored into the memory 28 is longer than a threshold value Th7 or not (step S206). When the elapsed time period "t12" is equal to or shorter than the threshold value Th7 (No in step S206), the operation of the breath analyzer 10 is returned to step S205. In contrast, when the elapsed time period "t12" is longer than the threshold value Th7 (Yes in step S206), the aberrance determination device 103a determines that an abnormal pressure occurs, and outputs information showing detection of the abnormal pressure to the display information generator 106. Receiving the information showing the detection of the abnormal pressure, the display information generator 106 displays a message showing detection of the abnormal pressure on the display device 11 a (step S209).

When the output value of the pressure sensor 16 is equal to or smaller than the threshold value Th4 (Yes in step S205), the aberrance determination device 103a stores the timing "tb" at which the output value of the pressure sensor 16 becomes equal to or smaller than the threshold value Th4 into the memory 28 (step S207).

Subsequently, the aberrance determination device 103a determines whether the first variation time period defined by "tb-ta" is shorter than the threshold value Th5 or not (step S208). When the first variation time period is shorter than the threshold value Th5 (Yes in step S208), the aberrance determination device 103a determines that no aberrance occurs, and the operation controller 102 activates the gas sensor 15 to sense alcohol in the breath sucked into the gas sensing chamber 15a. In contrast, when the first variation time period is equal to or longer than the threshold value Th5 (No in step S208), the aberrance determination device 103a determines that aberrance occurs, and outputs information showing detection of the abnormal pressure to the display information generator 106. Receiving the information showing the detection of the abnormal pressure, the display information generator 106 displays a message showing detection of the abnormal pressure on the display device 11a (step S209).

According to the second embodiment, when the first variation time is equal to or longer than the threshold value Th5, the aberrance determination device 103a determines that aberrance occurs. Therefore, it is possible to determine the occurrence of injustice such as jamming of the wadding or the like into the introduction opening 21a of the receptacle 20. In addition, if the dynamic range of the pressure sensor 16 is small, the output value of the pressure sensor 16 which is varied responding to the variation of the capacity of the receptacle 20 (or the bellows 17) by the movement of the solenoid 18 may be saturated and the occurrence or non-occurrence of aberrance may not be determined by the breath analyzer 10 in accordance with the above mentioned first embodiment. In contrast, in the second embodiment, even if the dynamic range of the pressure sensor 16 is small and the output value of the pressure sensor 16 is saturated, the occurrence or non-occurrence of aberrance can be determined by the breath analyzer 10.

### (Third Embodiment)

In the above mentioned first and second embodiments, the capacity of the receptacle 20 is decreased by expanding the plunger of the solenoid 18 first, and then, the capacity of the receptacle 20 is increased by contraction of the plunger of the solenoid 18, so that the breath is sucked into the receptacle 20 and the concentration of alcohol in the breath is measured. On the other hand, in the third embodiment, the capacity of the receptacle 20 is increased by contraction of the plunger of the solenoid 18 first, so that breath is sucked into the receptacle 20 and the concentration of alcohol in the breath is measured, and then, the breath in the receptacle 20 is exhausted by decreasing the capacity of the receptacle 20 by expanding the plunger of the solenoid 18. Subsequently, the output value of the pressure sensor 16 is compared with a threshold value Th8 when the capacity of the receptacle 20 is increased by contraction of the plunger of the solenoid 18 again. When the output value of the pressure sensor 16 is smaller than the threshold value Th8, it is determined that aberrance occurs. The threshold value Th8 satisfies the relation of Th8 < Th1.

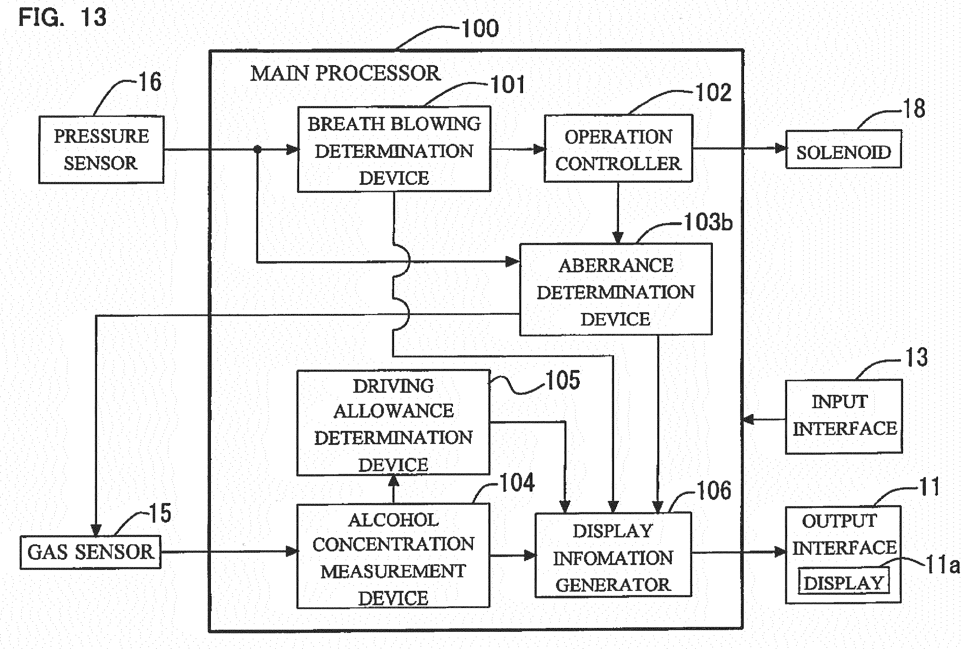

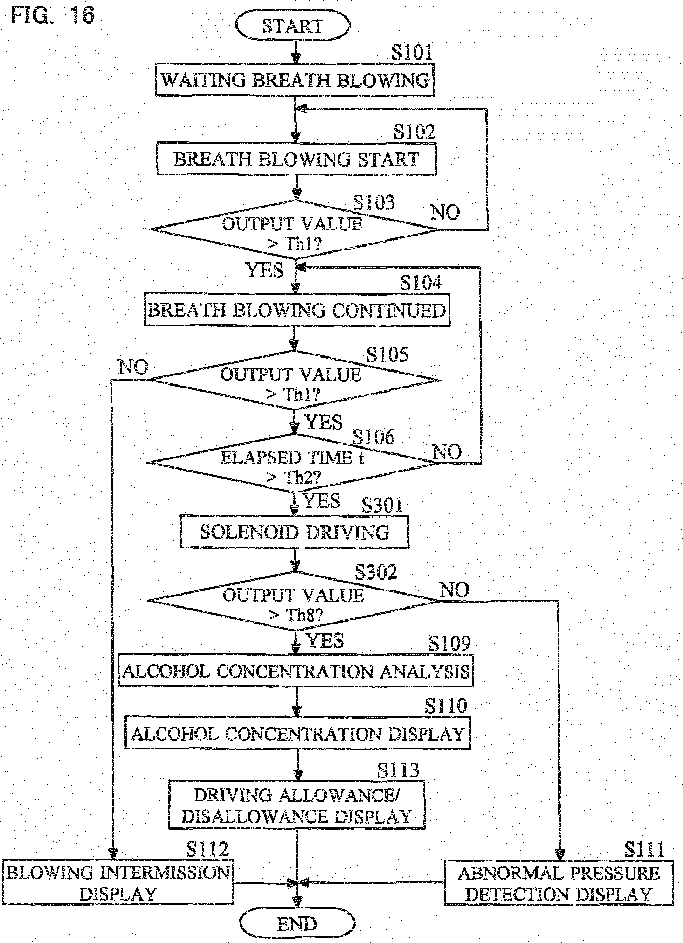

A constitution of the breath analyzer 10 in accordance with the third embodiment is different from that in the first embodiment at the points that the memory 28 stores a control program for controlling various operations of the main processor 100 such as a flow shown in FIG. 16 and the threshold value Th8, instead of the control program such as the flow shown in FIG. 8 and the threshold value Th3, and determination process in the aberrance determination device 103b in FIG. 13 is different from that in the above mentioned aberrance determination device 103 in FIG. 5.

FIG. 13 shows a functional block of the main processor 100 in the third embodiment. In the third embodiment, the main processor 100 reads out the control program stored in the memory 28 and executes the control program so as to serve as a breath blowing determination device 101, an operation controller 102, an aberrance determination device 103b, an alcohol concentration measurement device 104, an driving allowance determination device 105, a display information generator 106.

The aberrance determination device 103b determines that aberrance occurs, in the case where the output value of the pressure sensor 16 is smaller than the threshold value Th8 when the capacity of the receptacle 20 is increased by contraction of the plunger of the solenoid 18.

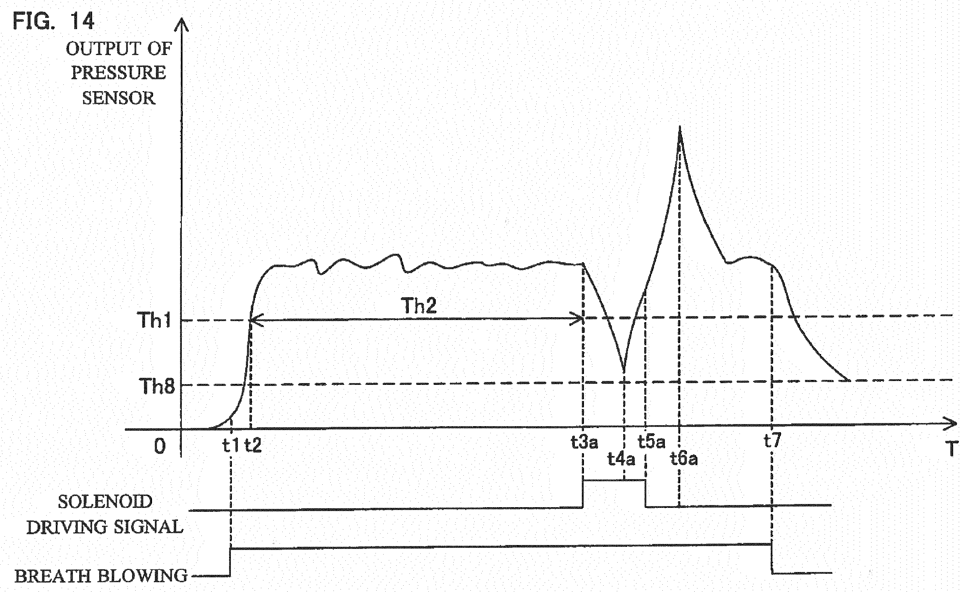

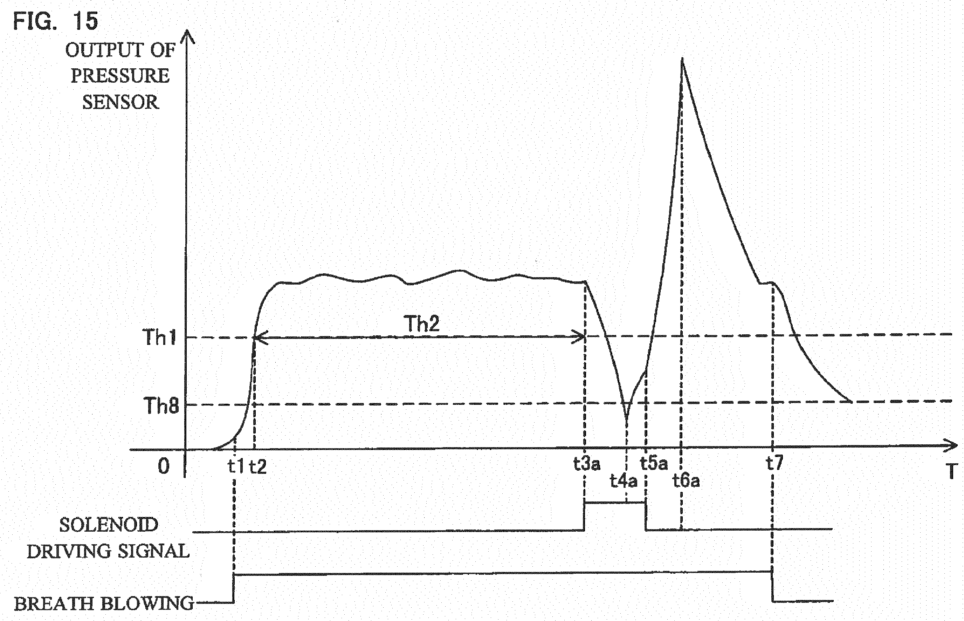

FIG. 14 shows an example of the variation of the output of the pressure sensor 16 (in other words the variation of the pressure in the receptacle 20) when the capacity of the receptacle 20 is once increased and subsequently decreased by driving the solenoid 18 under the state that noting is jammed in the introduction opening 21a of the receptacle 20. FIG. 15 shows an example of the variation of the output of the pressure sensor 16 when the capacity of the receptacle 20 is once increased and subsequently decreased by driving the solenoid 18 under the state that wadding is jammed in the introduction opening 21a of the receptacle 20.

In FIGS. 14 and 15, symbol "t3a" shows the timing at which an elapsed time period that the output value of the pressure sensor 16 is larger than the threshold value Th1 becomes longer than the threshold value Th2 and the operation controller 102 starts to output the solenoid control signal. In the third embodiment, the solenoid 18 starts to contract the plunger to expand the bellows 17 responding to the solenoid control signal so that the capacity of the bellows 17 (or the receptacle 20) increases. Symbol "t4a" shows the timing at which the expansion of the bellows 17 by the motion of the solenoid 18 is completed. Symbol "t5a" shows the timing at which the operation controller 102 stops to output the solenoid control signal. The solenoid 18 starts to expand the plunger to contract the bellows 17 when the solenoid control signal cannot be received, so that the capacity of the bellows 17 is decreased. Symbol "t6a" shows the timing at which contraction of the bellows 17 by the motion of the solenoid 18 is completed.

Subsequently, the operation of the breath analyzer 10 in accordance with the third embodiment is described with reference to the flowchart shown in FIG. 16. In FIG. 16, the same processes as those in FIG. 8 are designated by the same symbols. Differences from FIG. 8 are described below.

When the elapsed time period "t" is longer than the threshold value Th2 (Yes in step S106), the breath blowing determination device 101 outputs information showing starting of the sucking of the breath to the operation controller 102. When receiving the information showing starting of the sucking of the breath, the operation controller 102 outputs the solenoid control signal to the solenoid 18 for a constant time period so as to drive the solenoid 18 that the capacity of the receptacle 20 (or the bellows 17) is once increased and then decreased (step S301). When starting to output the solenoid control signal, the operation controller 102 outputs the driving information to the aberrance determination device 103b.

When wadding is jammed into the introduction opening 21 a, the sectional area of the introduction opening 21a becomes narrower, so that the output value of the pressure sensor 16 becomes equal to or smaller than the threshold value Th8 responding to the increase of the capacity of the receptacle 20 (or the bellows 17) as shown in FIG. 15. In contrast, when nothing is jammed into the introduction opening 21 a, the output value of the pressure sensor 16 becomes larger than the threshold value Th8 even when the capacity of the receptacle 20 (or the bellows 17) is increased as shown in FIG. 14.

When a second specified time period (for example a time period from the timing "t3a" to the timing "t4a" in FIGS. 14 and 15) has passed from the timing at which the aberrance determination device 103b receives the driving information, the aberrance determination device 103 b determines whether the output value of the pressure sensor 16 is larger than the threshold value Th8 or not (step S302). When the output value of the pressure sensor 16 is equal to or smaller than the threshold value Th8 (No in step S302), the aberrance determination device 103b determines that wadding is jammed into the introduction opening 21a and outputs information showing detection of abnormal pressure to the display information generator 106. In contrast, in the case where the output value of the pressure sensor 16 is larger than the threshold value Th8 when the second specific time period has passed from the timing at which the aberrance determination device 103b receives the driving information (Yes in step S302), the aberrance determination device 103b determines that nothing is jammed into the introduction opening 21a and the operation controller 102 activates the gas sensor 15.

According to the third embodiment, when the output value of the pressure sensor 16 is equal to or smaller than the threshold value Th8 when the capacity of the receptacle 20 (or the bellows 17) is increased, the aberrance determination device 103b determines that aberrance occurs. Therefore, it is possible to determine the occurrence of injustice such as jamming of the wadding in the introduction opening 21a.

### (Fourth Embodiment)

In a breath analyzer 10 in accordance with a fourth embodiment of the present invention, the capacity of the receptacle 20 is increased by contracting the plunger of the solenoid 18 first, so that the breath is sucked into the receptacle 20 and the concentration of alcohol in the breath is measured, and then, the capacity of the receptacle 20 is decreased by expanding of the plunger of the solenoid 18, and the breath in the receptacle 20 is exhausted, similar to the above mentioned third embodiment. Then, the operation to increase the capacity of the receptacle 20 is carried out, and occurrence or non-occurrence of aberrance is determined based on a time period from a timing at which the output value of the pressure sensor 16 becomes smaller than a threshold value Th9 to a timing at which the output value of the pressure sensor 16 becomes equal to or larger than the threshold value Th9. The threshold value Th9 satisfies the relation Th8 < Th9 < Th1.

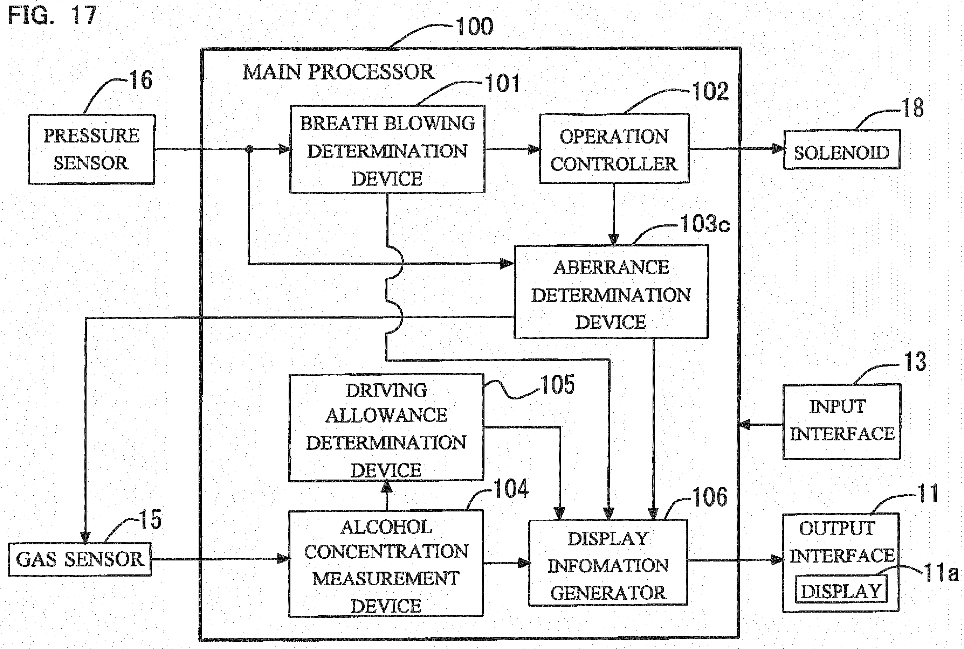

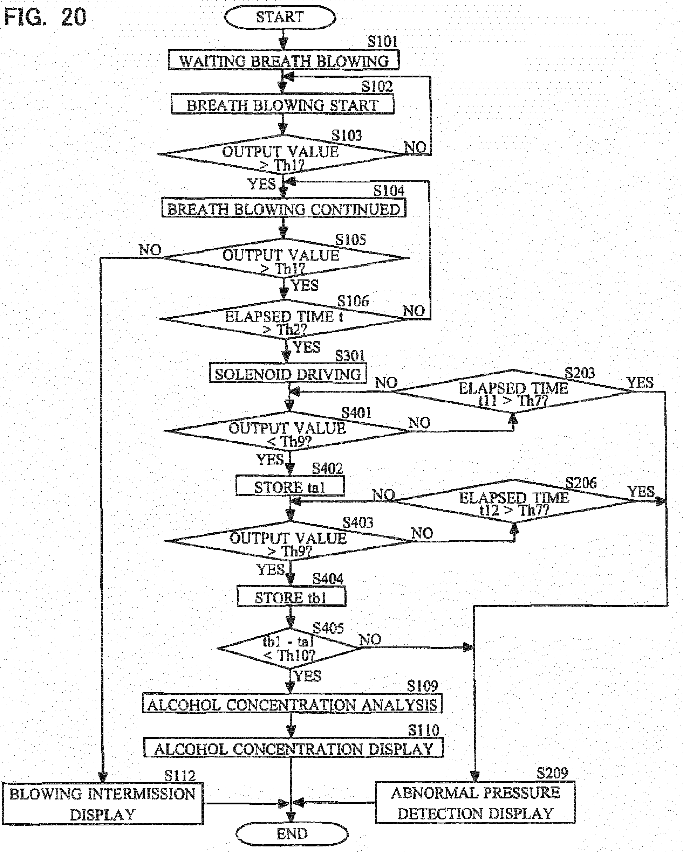

A constitution of the breath analyzer 10 in accordance with the fourth embodiment is different from that in the second embodiment at the points that the memory 28 stores a control program for controlling various operations of the main processor 100 such as a flow shown in FIG. 20 and the threshold values Th9 and Th10, instead of the control program such as the flow shown in FIG. 12 and the threshold values Th4 to Th6, and determination process in the aberrance determination device 103c in FIG. 17 is different from that in the above mentioned aberrance determination device 103a in FIG. 9.

FIG. 17 shows a functional block of the main processor 100 in the fourth embodiment. In the fourth embodiment, the main processor 100 reads out the control program stored in the memory 28 and executes the control program so as to serve as a breath blowing determination device 101, an operation controller 102, an aberrance determination device 103c, an alcohol concentration measurement device 104, a driving allowance determination device 105, a display information generator 106.

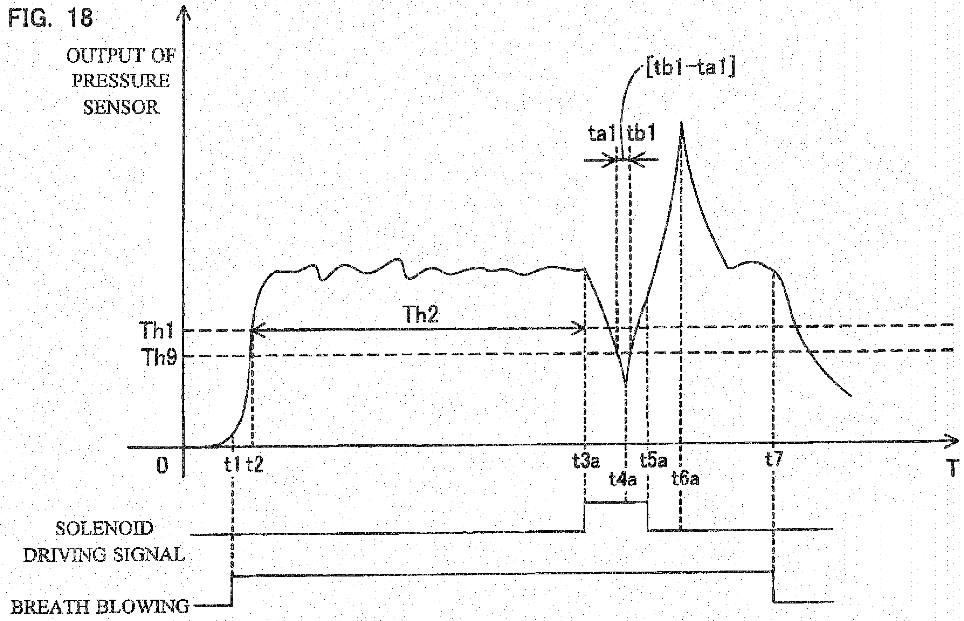

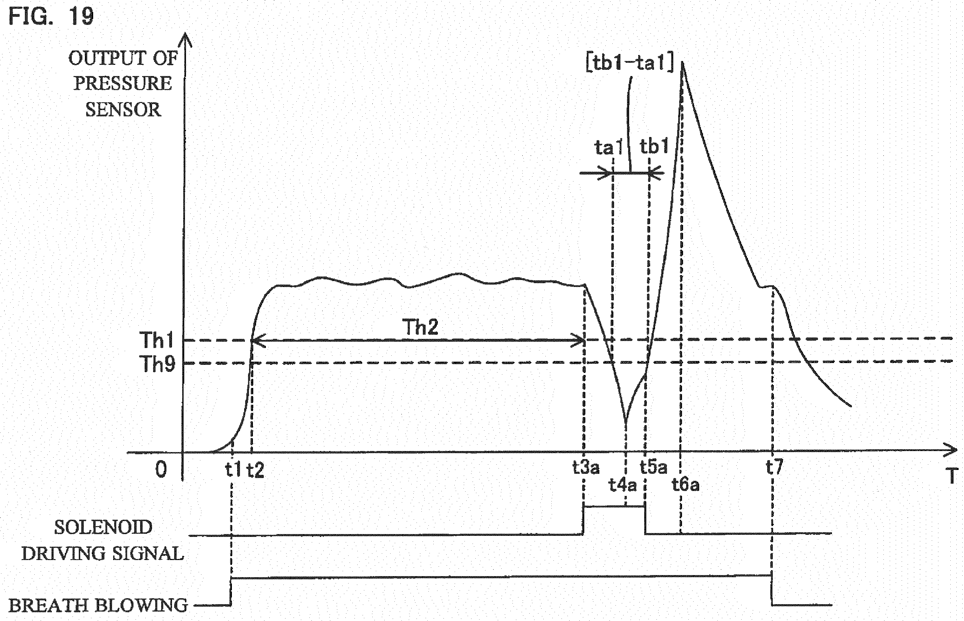

FIG. 18 shows an example of the variation of the output of the pressure sensor 16 when the capacity of the receptacle 20 is once increased and subsequently decreased by driving the solenoid 18 under the state that noting is jammed in the introduction opening 21a of the receptacle 20, similar to FIG. 14. FIG. 19 shows an example of the variation of the output of the pressure sensor 16 when the capacity of the receptacle 20 is once increased and subsequently decreased by driving the solenoid 18 under the state that wadding is jammed in the introduction opening 21a of the receptacle 20, similar to FIG. 15.

In FIGS. 18 and 19, symbol "ta1" shows the timing at which the output value of the pressure sensor 16 becomes smaller than the threshold value Th9 during the increase of the capacity of the receptacle 20. Symbol "tb1" shows the timing at which the output value of the pressure sensor 16 becomes equal to or larger than a threshold value Th9 after completion of the increase of the capacity of the receptacle 20.

The fewer the flow rate of gas (breath) per a unit time which goes into and out from the receptacle 20 becomes, the longer the time period defined by "tb1-ta1" (hereinafter it is called "second variation time period") becomes. The flow rate of gas becomes fewer responding to the narrowness of the sectional area of the introduction opening 21 a of the receptacle 20. Therefore, the narrower the sectional area of the introduction opening 21 a becomes, the longer the second variation time period becomes. Accordingly, if wadding is jammed into the introduction opening 21a and thus the sectional area of the introduction opening 21a becomes narrower, the second variation time period becomes longer than that when nothing is jammed in the introduction opening 21 a. Thus, the aberrance determination device 103 c determines that aberrance occurs when the second variation time period is equal to or longer than a threshold value Th10, and determines that no aberrance occurs when the second variation time period is shorter than the threshold value Th10. The threshold value Th9 corresponds to the sixth threshold value and the seventh threshold value in claim and the threshold value Th10 corresponds to the eighth threshold value in claim.

Subsequently, the operation of the breath analyzer 10 in accordance with the fourth embodiment is described with reference to the flowchart shown in FIG. 20. In FIG. 20, the same processes as those in FIG. 12 or 16 are designated by the same symbols. Differences from FIG. 12 or 16 are described below.

When the elapsed time period "t" is longer than the threshold value Th2 (Yes in step S106), the breath blowing determination device 101 outputs information showing starting of the sucking of the breath to the operation controller 102. Receiving the information showing starting of the sucking of the breath, the operation controller 102 outputs the solenoid control signal to the solenoid 18 for a constant time period so as to drive the solenoid 18 that the capacity of the receptacle 20 (or the bellows 17) is once increased and then decreased (step S301). Subsequently, the operation controller 102 outputs the driving information to the aberrance determination device 103c.

When receiving the driving information, the aberrance determination device 103c determines whether the output value of the pressure sensor 16 is smaller than the threshold value Th9 or not (step S401). When the output value of the pressure sensor 16 is equal to or larger than the threshold value Th9 (No in step S401), the aberrance determination device 103c determines whether the elapsed time period t11 is longer than a threshold value Th7 or not (step S203). When the elapsed time period t11 is equal to or shorter than a threshold value Th7 (No in step S203), the operation of the breath analyzer 10 is returned to the step S401. In contrast, when the elapsed time period t11 is longer than a threshold value Th7 (Yes in step S203), the display information generator 106 displays a message showing the detection of abnormal pressure on the display device 11a (step S209). On the other hand, when the output value of the pressure sensor 16 is equal to or smaller than the threshold value Th9 (Yes in step S401), the aberrance determination device 103c stores the timing "ta1" into the memory 28 (step S402).

Subsequently, the aberrance determination device 103c determines whether the output value of the pressure sensor 16 is larger than the threshold value Th9 or not (step S403). When the output value of the pressure sensor 16 is equal to or smaller than the threshold value Th9 (No in step S403), the aberrance determination device 103c determines whether an elapsed time period t12 from a timing at which the timing "ta1" is stored in the memory 28 is longer than a threshold value Th7 or not (step S206). In contrast, when the output value of the pressure sensor 16 is larger than the threshold value Th9 (Yes in step S403), the aberrance determination device 103c stores the timing "tb1" into the memory 28 (step S404).

Subsequently, the aberrance determination device 103c determines whether the second variation time period defined by "tb1-ta1" is shorter than the threshold value Th10 or not (step S405). When the second variation time period is shorter than the threshold value Th10 (Yes in step S405), the aberrance determination device 103 c determines that no aberrance occurs, and the operation controller 102 activates the gas sensor 15. In contrast, when the second variation time period is equal to or longer than the threshold value Th10 (No in step S405), the aberrance determination device 103 c determines that aberrance occurs, and outputs information showing detection of abnormal pressure to the display information generator 106. Then, the display information generator 106 displays a message showing the detection of abnormal pressure on the display device 11a (step S209).

According to the fourth embodiment, when the second variation time period is equal to or longer than the threshold value Th10, the aberrance determination device 103c determines that aberrance occurs. Therefore, it is possible to determine the occurrence of injustice such as jamming of the wadding or the like in the introduction opening 21 a of the receptacle 20.

The threshold values used in the above mentioned embodiments are preferably set arbitrarily responding to operation characteristics of the breath analyzer 10.

### (Modified Examples)

The present invention is not limited to the above mentioned embodiments, and it is possible to modify the invention in various manners as mentioned below. In addition, it is further possible to combine at least two modified examples optionally selected among the following modified examples.

### (First Modified Example)

In the third embodiment, the memory 28 may store the threshold value Th3 instead of the threshold value Th8, and the aberrance determination device 103b may determine the occurrence of aberrance when the output value of the pressure sensor 16 detected during the decreasing of the capacity of the receptacle 20 is equal to or larger than the threshold value Th3.

In the fourth embodiment, the memory 28 may store the threshold values Th4 and Th5 instead of the threshold values Th9 and Th10, and the aberrance determination device 103c may determine the occurrence of aberrance when the first variation time period is equal to or longer than the threshold value Th5.

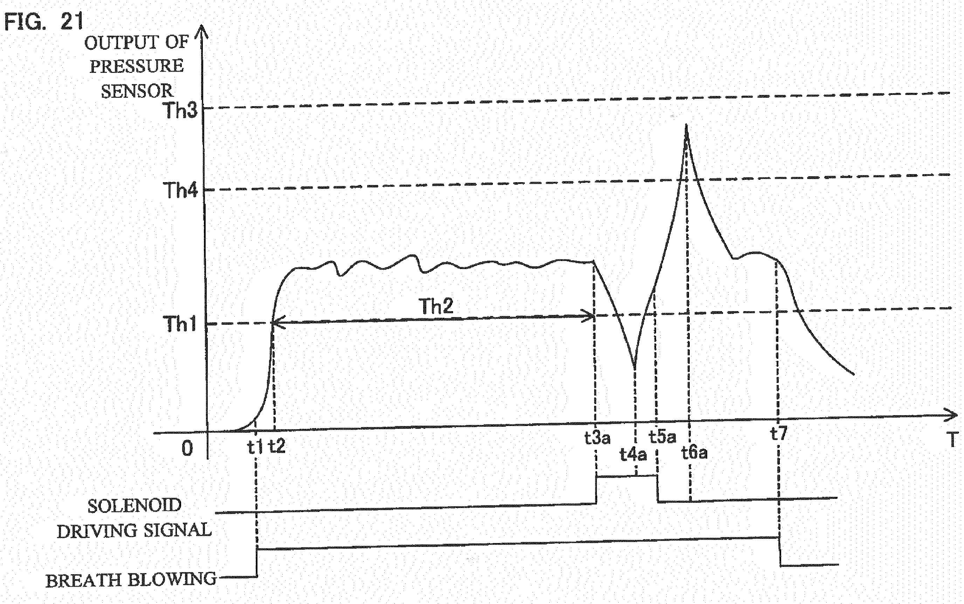

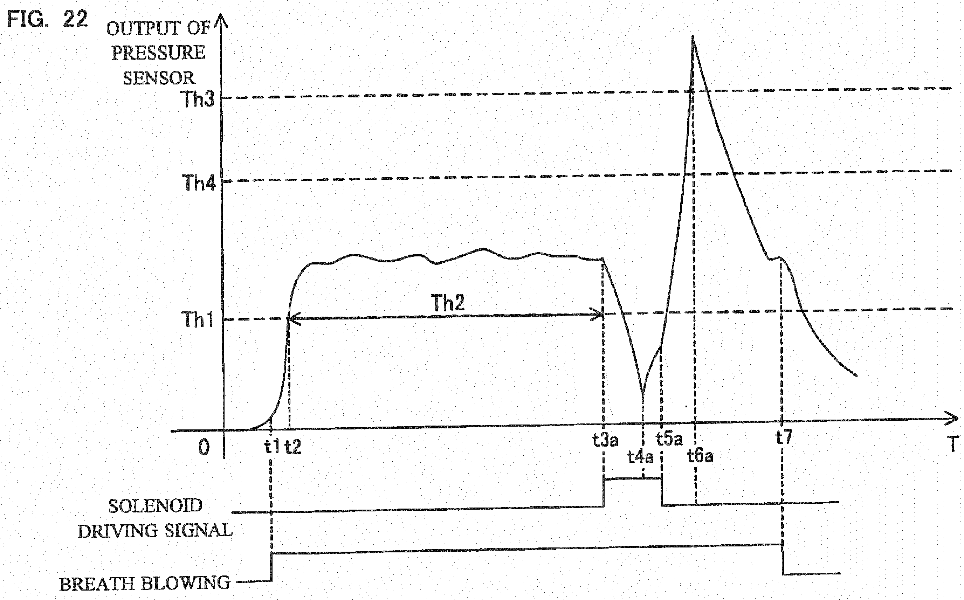

FIG. 21 shows an example of the variation of the output of the pressure sensor 16 when the capacity of the receptacle 20 is once increased and subsequently decreased by driving the solenoid 18 under the state that noting is jammed in the introduction opening 21 a with the threshold values Th3 and Th4. FIG. 22 shows an example of the variation of the output of the pressure sensor 16 when the capacity of the receptacle 20 is once increased and subsequently decreased by driving the solenoid 18 under the state that wadding is jammed in the introduction opening 21a with the threshold values Th3 and Th4. The threshold value Th3 used in the first modified example may be a different value from that used in the first embodiment. Similarly, the threshold value Th4 used in the first modified example may be a different value from that used in the second embodiment.

### (Second Modified Example)

In the second embodiment, the threshold value (second threshold value) used in the step S202 and the threshold value (third threshold value) used in the step S205 are the same threshold value Th4. However, the second threshold value may be different from the third threshold value.

In the fourth embodiment, the threshold value (sixth threshold value) used in the step S401 and the threshold value (seventh threshold value) used in the step S403 are the same threshold value Th4. However, the sixth threshold value may be different from the seventh threshold value.

### (Third Modified Example)

The aberrance determination device 103 may determine whether the output value of the pressure sensor 16 is equal to or smaller than the threshold value Th3 or not in the step S108 of the flow chart in FIG. 8. Similarly, the abnormal blow determination device 107 may determine whether the output value of the pressure sensor 16 is equal to or larger than the threshold value Th6 or not in the step S201 of the flow chart in FIG. 12. In addition, the aberrance determination device 103a may determine whether the output value of the pressure sensor 16 is equal to or larger than the threshold value Th4 or not in the step S202. In addition, the aberrance determination device 103a may determine whether the output value of the pressure sensor 16 is equal to or smaller than the threshold value Th4 or not in the step S205. In addition, the aberrance determination device 103a may determine whether the first variation time period defined by "tb-ta" (first variation time period) is equal to or shorter than the threshold value Th5 or not in the step S208. In addition, the aberrance determination device 103b may determine whether the output value of the pressure sensor 16 is equal to or larger than the threshold value Th8 or not in the step S302 of the flow chart in FIG. 16. In addition, the aberrance determination device 103 c may determine whether the output value of the pressure sensor 16 is equal to or smaller than the threshold value Th9 or not in the step S401 of the flow chart in FIG. 20. In addition, the aberrance determination device 103c may determine whether the output value of the pressure sensor 16 is equal to or larger than the threshold value Th9 or not in the step S403. In addition, the aberrance determination device 103c may determine whether the second variation time period defined by "tb1-ta1" (second variation time period) is equal to or shorter than the threshold value Th10 or not in the step S405.

### (Fourth Modified Example)

Each functions of the breath blowing determination device 101, the operation controller 102, the aberrance determination device 103, 103a, 103b and 103c, the alcohol concentration measurement device 104, the driving allowance determination device 105, and the display information generator 106, the abnormal blow determination device 107, and so on, which are realized by performing the control program in the main processor 100, may be realized by hardware such as electric circuits.

### (Fifth Modified Example)

The abnormal blow determination device 107 used in the second embodiment may be used in the first embodiment, too. In such a case, it is preferable that the abnormal blow determination device 107 determines that abnormal blowing occurs when the output value of the pressure sensor 16 is larger than the threshold value Th3 responding to the blowing of the breath into the breath flow path 33.

### (Sixth Modified Example)

In the above mentioned embodiments, although alcohol in the breath is measured as an example of the predetermined component in the breath, the breath analyzer 10 in accordance with the embodiments of the present invention may measure another component in the breath.

### (Seventh Modified Example)

The output interface 11 for outputting information such as a message corresponding to result of determination by the aberrance determination device 103, 103a, 103b or 103c is not limited to the display device 11a. For example, a transmitter to transmit the information showing the determination result to communication terminal equipment or a server may be used as the output interface 11.

## Claims

1. A breath analyzer (10) comprising:
a receptacle (20) having an introduction opening (21 a) through which a breath of a subject is introduced and containing the breath introduced through the introduction opening;
a breath component sensor (15) for sensing a predetermined component in the breath in the receptacle;
a pressure sensor (16) for sensing a pressure in the receptacle;
a capacity varying device (18) for varying a capacity of the receptacle; and
an aberrance determination device (103) for determining whether aberrance occurs or not, based on an output of the pressure sensor responding to variation of the capacity of the receptacle by the capacity varying device.

2. The breath analyzer (10) in accordance with claim 1, wherein
the aberrance determination device (103) determines that aberrance occurs when the output value of the pressure sensor (16) responding to decreasing of a capacity of the receptacle (20) by the capacity varying device (18) is larger than a first threshold value (Th3).

3. The breath analyzer (10) in accordance with claim 1, wherein
the aberrance determination device (103) determines that aberrance occurs when a time period from a timing at which the output value of the pressure sensor (16) responding to decreasing of a capacity of the receptacle becomes larger than a second threshold value (Th4) to a timing at which the output value of the pressure sensor after completion of the decreasing of the capacity of the receptacle becomes smaller than a third threshold value (Th4) is longer than a fourth threshold value (Th5).

4. The breath analyzer (10) in accordance with claim 1, wherein
the aberrance determination device (103) determines that aberrance occurs when the output value of the pressure sensor responding to increase of a capacity of the receptacle becomes smaller than a fifth threshold value (Th8).

5. The breath analyzer (10) in accordance with claim 1, wherein
the aberrance determination device (103) determines that aberrance occurs when a time period from a timing at which the output value of the pressure sensor responding to increasing of a capacity of the receptacle becomes smaller than a sixth threshold value (Th9) to a timing at which the output value of the pressure sensor after completion of the increase of the capacity of the receptacle becomes larger than a seventh threshold value (Th9) is longer than an eighth threshold value (Th10).

6. The breath analyzer (10) in accordance with one of claims 1 to 3, further comprising:
a breath flow path (33) into which the breath is blown by the subject and which is communicated to the introduction opening (21 a); and
an abnormal blow determination device (107) for determining that abnormal blowing occurs when the output value of the pressure sensor during the subject's blow of the breath into the breath flow path is larger than a ninth threshold value (Th6).

7. The breath analyzer (10) in accordance with one of claims 1 to 6, further comprising:
an output device (11a) for outputting information corresponding to result of determination by the aberrance determination device.

8. A method for determining aberrance in a breath analyzer (10) determining whether aberrance in breath component measurement occurs or not, having the steps of:
introducing breath of a subject into a receptacle (20) of the breath analyzer through an introduction opening (21a);
sensing a predetermined component in the breath in the receptacle;
varying a capacity of the receptacle; and
determining whether aberrance occurs or not, based on an output of a pressure sensor (16) responding to variation of the capacity of the receptacle.
